**Europäisches Patentamt**

**European Patent Office**

**Office européen des brevets**

(19)

(11) Veröffentlichungsnummer: **0 679 405 A1**

## (12) EUROPÄISCHE PATENTANMELDUNG

(21) Anmeldenummer: **94810231.4**

(51) Int. Cl.[6]: **A61L 2/00**

(22) Anmeldetag: **25.04.94**

(43) Veröffentlichungstag der Anmeldung:
**02.11.95 Patentblatt 95/44**

(84) Benannte Vertragsstaaten:
**AT BE CH DE DK ES FR GB GR IE IT LI LU MC NL PT SE**

(71) Anmelder: **ROTKREUZSTIFTUNG ZENTRALLABORATORIUM BLUTSPENDEDIENST SRK**
**Wankdorfstrasse 10**
**CH-3000 Bern 22 (CH)**

(72) Erfinder: **Omar, Adames**
**Engestr. 11**
**3012 Bern (CH)**
Erfinder: **Moergenthaler, Jean-Jacques**
**Aeschiweg 9**
**3067 Boll (CH)**

(74) Vertreter: **Fischer, Franz Josef et al**
**BOVARD AG**
**Patentanwälte VSP**
**Optingenstrasse 16**
**CH-3000 Bern 25 (CH)**

(54) **Verfahren zur Abtrennung von Viren aus Proteinlösungen.**

(57) Zur Abtrennung von Viren aus Proteinlösungen werden darin Partikel einer festen Phase zwecks Adsorption der Viren aufgeschlämmt oder suspendiert. Die feste Phase ist ein Adsorbens, das aus der Gruppe Kieselgur, Kieselsäure, Tonmineralien, Metallhydroxid oder -oxihydrat, Cellulose, Perlit und wasserunlösliche synthetische Polymere ausgewählt ist. Anschliessend wird die feste Phase mit den daran adsorbierten Viren von der flüssigen Phase abgetrennt. Die Proteinlösung wird hierzu mindestens einmal mit der festen Phase während mindestens 10 min. unter Bildung einer Suspension oder Aufschlämmung in Kontakt gebracht und anschliessend wieder vom Adsorbens getrennt.

EP 0 679 405 A1

Die vorliegende Erfindung betrifft ein Verfahren zur Abtrennung von Viren aus Proteinlösungen, insbesondere aus Blutprodukten, die für therapeutische Zwecke und andere medizinische Zwecke bestimmt sind. Dabei wird die Proteinlösung mit einem Adsorbens in Kontakt gebracht, an welchem die Viren gebunden werden.

Im zweiten Weltkrieg wurde in den U.S.A. ein Verfahren entwickelt welches es erlaubt, aus menschlichem Blutplasma Proteine zu isolieren, die in der Medizin als Therapeutika eingesetzt werden können. Beispiele solcher Proteine sind Albumin, Immunglobuline, Fibrinogen, Gerinnungsfaktoren, und zahlreiche andere. Albumin wird beispielsweise bei Verbrennungen eingesetzt, oder allgemein bei Zuständen, in denen das Blutvolumen vergrössert werden muss; Immunglobuline können u.a. bei Patienten verwendet werden, welche selber nicht imstande sind, schützende Antikörper in ihrem Körper zu synthetisieren; Gerinnungsfaktoren (besonders Faktor VIII und Faktor IX) werden bei Hämophilie-Patienten gebraucht. In vielen Fällen sind diese Präparate lebensrettend und deshalb kann auf ihre Verwendung nicht verzichtet werden.

Die Verfahren zur Trennung des Blutplasmas in einzelne Proteine beruhen auf verschiedenen Prinzipien. Die älteren Verfahren - welche aber immer noch im grossen Massstab verwendet werden - beruhen auf der fraktionierten Fällung der Proteine mit Alkohol und anschliessender Trennung der Phasen durch Zentrifugation oder Filtration. Neuere Verfahren verwenden auch andere Trennmethoden, wie z.B. Ionentauschchromatographie oder (Immun-)Affinitätschromatographie. Ein integriertes Trennschema umfasst meist verschiedene Trennmethoden in einem optimierten Gesamtverfahren.

Schon in den ersten Jahren der Anwendung von Plasmaproteinen an Patienten wurde es klar, dass diese aus menschlichem Blut isolierte Produkte auch Nachteile haben: sie können unter ungünstigen Umständen gewisse infektiöse Krankheiten übertragen, welche durch Viren verursacht werden. Zunächst handelte es sich dabei um virale Hepatitis (Hepatitis B); später kam *non−A, non−B* Hepatitis dazu (deren Erreger, oder zumindest der Erreger der meisten Fälle von *non−A, non−B* Hepatitis, wurde kürzlich identifiziert und als Hepatitis C Virus bezeichnet). Allgemein bekannt - und gefürchtet - ist die Übertragung von HIV (human immunodeficiency virus), dem Erreger von AIDS (acquired immuno deficiency syndrome), durch Blut und Blutprodukte. Abgesehen von den bisher erwähnten gibt es noch einige andere Viren, die mit mehr oder weniger grosser Wahrscheinlichkeit durch Plasma und Plasmaderivate übertragen werden können.

Die Sicherheit von Blut und Blutderivaten kann durch verschiedene Massnahmen erhöht werden: (1 ) Es wird danach getrachtet, Blutspender oder Blutspenderinnen, die bekanntermassen ein erhöhtes Risiko aufweisen, von der Blutspende auszuschliessen. Dies erfolgt mit Hilfe eines Fragebogens, der es erlaubt, Personen mit erhöhten Risiken auszuschliessen. Erhöhte Risiken haben beispielsweise Personen, welche an bestimmten Krankheiten leiden, welche sich kurz vor der Blutspende in gewissen Ländern aufhielten, solche mit risikoreichem sexuellem Verhalten, oder Drogensüchtige, welche kontaminerte Nadeln benützen. (2) Analysen im Laboratorium erlauben es, infektiöse Spenden zu erfassen und der weiteren Verteilung zu entziehen. Diese beiden Massnahmen zusammen erlauben zwar, die meisten infektiösen Spenden zu eliminieren, aber nicht restlos alle: (a) Die Empfindlichkeit der Testmethoden kann ungenügend sein; (b) für ein Virus ist ein Test (noch) nicht erhältlich; aus praktischen und wirtschaftlichen Gründen kann ausserdem nicht auf alle denkbaren Viren geprüft werden; (c) der Test erfasst nicht das Virus, sondern die Antikörper, welche von der infizierten Person erst als Antwort auf die Virusinfektion gemacht werden; vom Moment der Infektion bis zur Bildung von nachweisbaren Antikörpern verstreichen aber meist einige Tage bis Wochen (sogenanntes " Fenster"); Antikörperteste sind in dieser Zeit nutzlos; (d) durch Fehler in der Buchhaltung wird irrtümlich eine infizierte Spende freigegeben. (3) Aus diesen Gründen ist es wichtig, dass während der Produktion von Blut oder Blutbestandteilen aktiv die Sicherheit bezüglich Übertragung von Viren erhöht wird. Dies geschieht durch spezielle, in den Prozess eingeführte Schritte.

Für zellhaltige Blutprodukte (Erythrozyten- und Plättchenkonzentrate) werden solche Schritte gegenwärtig entwickelt; sie sind aber noch nicht produktionsreif. Isolierte Plasmaproteine hingegen werden schon seit mehreren Jahren speziellen Behandlungen unterworfen, welche Viren eliminieren oder inaktivieren. Eines der ältesten Verfahren ist das Pasteurisieren (Erhitzen der Lösung während 10 oder mehr Stunden auf eine Temperatur von mindestens 60°C); diese Methode wurde ursprünglich zur Inaktivierung von Hepatitis B Viren in Albuminlösungen entwickelt. Heute werden von bestimmten Herstellern auch Gerinnungsfaktorpräparate, Immunglobuline und andere Plasmaproteine durch Pasteurisation virenfrei gemacht.

In den vergangenen Jahren sind noch zahlreiche andere Methoden entwickelt worden, um die Sicherheit bezüglich Übertragung von Viren zu verbessern; es sind dies unter anderem: Erhitzen des lyophilisierten (gefriergetrockneten) Endprodukts auf 60 bis 80°C während 24 bis 72 Stunden; Behandeln der Proteinlösung mit $\beta$-Propiolakton und ultraviolettem Licht (führt zur Einführung von $\beta$-Hydroxypropionsäureresten in Proteine); Behandeln mit einer Kombination eines Lösungsmittels und eines Detergens (sogenannte S/D-Behandlung; zerstört die Lipidhülle von Viren); Belichten der Proteinlösung nach Zusatz eines geeigneten Farbstoffs (photodynamische Vireninaktivierung; führt zu Modifikation von Nukleinsäuren). Die verschiedenen Methoden zur Inaktivierung von Viren in Plasmaproteinpräparaten sind in verschiedenen Übersichtsarbeiten beschrieben worden (z.B. "Virus Inactivation in Plasma Products"; Current Studies In Hematology and Blood Transfusion; J.J. Morgenthaler, Herausgeber; Karger, Basel, 1989). Es ist allgemein bekannt, dass alle diese Verfahren auf behüllte Viren besser wirken als auf kleine, unbehüllte Viren.

Neben diesen eigentlichen Methoden zur Inaktivierung von Viren tragen Prozessschritte oft auch in anderer Weise zur Verbesserung der Virensicherheit bei: während der Plasmafraktionierung verteilen sich die eventuell vorhandenen Viren auf die verschiedenen Phasen; falls Viren und das (oder die) interessierende(n) Protein(e) in verschiedenen Phasen verteilt sind, resultiert daraus eine physikalische Abreicherung der Viren. So wurde gezeigt, dass beispielsweise HIV bei der Herstellung von Immunglobulinen durch fraktionierte Fällung mit Alkohol um viele Grössenordnungen abgereichert wird [M.A. Wells und Mitarb., Transfusion **26**, 210-213 1986]. Auch bei anderen Trennmethoden, wie z. B. bei der Immunaffinitätschromatographie wurde eine Abreicherung von Viren während der Isolierung der Proteine beobachtet [D. Piszkiewicz und Mitarb., Thrombosis Research **55**, 627-634, 1989].

Trotzdem besteht für Unternehmen, die sich mit der Verarbeitung von humanem Blut befassen, ein Bedarf nach industriell anwendbaren Methoden zur sicheren Abtrennung von Viren aus Proteinlösungen, um die Infektionsrisiken für Patienten, welche mit derartigen Produkten behandelt werden, zu vermindern. Es ist demzufolge Aufgabe der vorliegenden Erfindung ein Verfahren zur Verfügung zu stellen, welches die Abtrennung von Viren aus Proteinlösungen in industriellem Masstab ermöglicht und insbesondere in die geläufigen Plasmafraktionierungsverfahren eingebaut werden kann.

Völlig überraschend wurde nun gefunden, dass viele gegebenenfalls in Proteinlösungen vorhandene Viren (behüllte und unbehüllte) an Adsorbentien, wie modifizierte Zellulosen, Diatomeenerde, Bentonite, Vulkangestein, Kunststoffpartikel usw. adsorbiert werden, wenn die Proteinlösungen während genügend langer Zeit mit diesen Adsorbentien in Kontakt gebracht werden. Damit wurde erkannt, dass sich bei der Phasentrennung die Möglichkeit einer über die durch blosse Präzipitation bewirkte, weitere Abreicherung von Viren ergibt. Der Effekt wurde bei behüllten und bei unbehüllten Viren gleichermassen beobachtet. Dies obschon derartige Adsorbentien als sogenannte Filterhilfsmittel bei der schon erwähnten Filtration für die Trennung der Phasen bei der Alkoholfraktionierung von Plasmaproteinen zum Einsatz gelangen und diese Trennung durch Bildung einer Schicht auf den eigentlichen Filtern oftmals erst dadurch ermöglicht wird; indem diese Schicht die Flüssigkeit durchlässt, feste Teilchen aber nicht; dabei wird das Verstopfen der Filter durch kleine Proteinpartikel verhindert.

Erfindungsgemäss werden somit Viren aus proteinhaltigen Lösungen durch Adsorption auf eine feste Phase entfernt, indem Partikel der festen Phase zwecks Adsorption der Viren in der Lösung suspendiert oder aufgeschlämmt werden und anschliessend eine Trennung der festen Phase mit den daran adsorbierten Viren von der flüssigen Phase erfolgt, wobei die Proteinlösung mindestens einmal mit einer festen Phase in Form mindestens eines Adsorbens, ausgewählt aus der Gruppe Kieselgur, Kieselsäure, Tonmineralien, Metallhydroxid oder -oxihydrat, Cellulose, Perlit und wasserunlösliche synthetische Polymere, während mindestens 10 min. unter Bildung einer Suspension oder Aufschlämmung in Kontakt gebracht und anschliessend vom Adsorbens getrennt wird.

Als feste Phase kommen beispielsweise die als Filterhilfsmittel bekannten Produkte wie Celite® (Johns-Manville Corp.) Aerosil® (Degussa), Perlit, Puffperlit, Bentonite, oder allgemein feinverteilte Festkörper in Frage, welche durch Filtration oder Zentrifugation wiederum aus einer Suspension entfernt werden können. Auch Stoffe wie Metallhydroxidgele (z. B. Alhydrogel, $Al(OH)_3$ in Form eines wässerigen Gels) sind geeignet, wie in einem der Beispiele gezeigt wird.

Die Adsorption von Viren auf den erwähnten Materialien hängt ab vom Material, dem Virus und dem Milieu. Durch gezielte Änderungen des Milieus kann die "Klebrigkeit" eines bestimmten Virus auf ein und demselben Material verändert werden. So ist es beispielsweise möglich, durch Senken des pH-Wertes die Adsorption von Semliki Forest Virus, Sindbis Virus, vesikulärem Stomatitis Virus oder Coxsackie Virus an Celite 577® deutlich zu verbessern (siehe Beispiel 6). Auch andere Lösungsparameter wie Ionenstärke, Salze, organische Lösungsmittel beeinflussen die Adsorption von Viren und können benutzt werden, um diese Adsorption zu vergrössern und damit die Entfernung der Viren aus der Proteinlösung zu verbessern. Die Arbeitstemperatur für das Verfahren beträgt vorzugsweise 2 °C. Es kann auch bei andern Temperaturen, z.B. bei Zimmertemperatur, gearbeitet werden, sofern das zu behandelnde Produkt bei diesen Temperaturen stabil ist.

Schon bei einer Behandlung der Proteinlösung während 10 min konnte eine Abreicherung beobachtet werden. Es kann jedoch eine längere Behandlungszeit von Vorteil sein um eine grössere Abreicherung zu erzielen. Mögliche Behandlungszeiten sind: ca. 15 min, ca. 30 min, ca. 1 h, ca. 2 h, ca. 6 h, ca. 8 h, ca. 10 h, ca. 12 h, ca. 14 h, ca. 16 h, ca. 20 h.

Die erfindungsgemässe Abtrennung des Adsorbens mitsamt den eventuell vorhandenen Viren erfolgt, wie oben erwähnt, durch Zentrifugation oder Filtration. Zentrifugation kann auf verschiedenste Art und Weise durchgeführt werden, z.B. in einem batch-Verfahren oder mittels einer kontinuierlichen Zentrifuge. Zentrifugalbeschleunigung und Zentrifugationszeit müssen in jedem Fall so gehalten werden, dass eine saubere Trennung von suspendierten Stoffen und Überstand erfolgt. Filtration kann im Labormassstab mit einer Nutsche oder einem Filtertopf o.ä. durchgeführt werden; im grösseren Massstab (Produktion) werden andere Geräte bevorzugt, beispielsweise Filterpressen oder Drehfilter.

**Beispiel 1**

Zu je 50 ml Immunglobulin G Lösung wurde 1 ml Virensuspension mit bekanntem Titer zugegeben und gemischt. Ein Aliquot wurde entnommen und der Virustiter darin bestimmt. Dann wurden 0.1 g Celite 577 zugegeben und es wurde 15 Minuten bei Zimmertemperatur mit einem magnetischen Rührer gemischt. Anschliessend wurde durch ein Zellulosefilter AF9 filtriert. Im Filtrat wurden die Viren durch Titration bestimmt (in allen folgenden Tabellen ist "E+X" gleichbedeutend mit "$10^X$"):

| Virus | Titer vor Filtration | Titer nach Filtration | | Geometrisches Mittel | Abreicherungsfaktor |
|---|---|---|---|---|---|
| Semliki Forest virus (SFV) | 1.0E+10 | 5.8E + 6 | 3.0E+6 | 4.2E+6 | 2.4E+3 |
| Vesicular stomatitis virus (VSV) | 1.0E+10 | 4.8E+7 | 3.9E+7 | 5.4E+7 | 2.9E+2 |
| Bovine viral diarrhoea virus Pseudorabies virus | 1.0E+7 1.0E+7 | 9.3E+3 1.4E+3 | 6.8E+3 9.6E+3 | 8.0E+3 3.7E+3 | 1.3E+3 2.7E+3 |
| Sindbis virus | 1.0E+12 | 3.8E+8 | 3.6E+7 | 1.2E+8 | 8.5E+3 |
| Cocksackie virus | 1.0E+7 | <10 | | <10 | >1.0E+6 |
| Bovine Parvovirus (BPV) | 1.0E+7 | 3.2E+3 | 4.8E+3 | 3.9E+3 | 2.6E+3 |

Für alle untersuchten Viren ergaben sich somit Abreicherungen um mehrere Grössenordnungen.

**Beispiel 2**

Zu je 50 ml Immunglobulin G Lösung, pH 6.5, wurde 1 ml Virensuspension mit bekanntem Titer zugegeben und gemischt. Dann wurden 4.5 g Alhydrogel Suspension zugegeben und der pH wurde mit 0.1 N NaOH auf 7.2 eingestellt. Nach 2 Stunden Rühren mit einem Magnetrührer bei Zimmertemperatur wurde der pH mit 0.2 N HCl auf 6 gesenkt. Nach weiteren 12 Stunden Rühren wurde 1 g Celite 577® zugegeben und weitere 15 Minuten lang gerührt. Anschliessend wurde durch ein Zellulose Filter AF9 filtriert und die Viren wurden im Filtrat titriert:

| Virus | Titer vor Filtration | Titer nach Filtration | | Geomertrisches Mittel | Abreicherungsfaktor |
|---|---|---|---|---|---|
| SFV | 1.0E+10 | 4. 2E + 6 | 5.2E+6 | 4.7E+6 | 2.1E+3 |
| Sindbis | 1.0E+12 | 4.6E+7 | 3.9E+7 | 5.4E+7 | 1.8E+4 |
| VSV | 1.0E+10 | <10 | | <10 | >10E+8 |
| Coxsackie | 1.0E+7 | <10 | | <10 | >10E+6 |

Bei VSV und Coxsackie Virus fällt auf, dass die Abreicherung durch den Zusatz von Alhydrogel stark erhöht wurde.

**Beispiel 3**

Durch Einfrieren, nachfolgendes Auftauen bei 2 ° C und anschliessende Zentrifugation wurde aus Plasma das sogenannte Kryopräzipitat entfernt. Der Überstand nach dieser Behandlung (sogenannter Kryoüberstand) wurde für die weiteren Versuche verwendet. Zu 85 ml Kryoüberstand wurde 1 ml Hepatitis A Virus (HAV) mit einem Titer von $10^8$ $TCID_{50}/ml$ gegeben. Anschliessend wurden 0.43 g Perlite J100 und 1.28 g Celite 577 zugegeben und es wurde während 15 Minuten bei Zimmertemperatur gerührt. Dann wurde durch ein Zellulosefilter AF9 filtriert und im Filtrat wurde HAV titriert. Zwei unabhängige Bestimmungen ergaben total $6.3 \times 10^5$ und $2.5 \times 10^6$ $TCID_{50}$ (geometrisches Mittel: $1.2 \times 10^6$ $TCID_{50}$). Daraus berechnet sich eine Abreicherung von HAV um den Faktor $8 \times 10^1$.

**Beispiel 4**

Zu 85 ml einer Lösung von Plasmaproteinen wurde 1 ml einer HAV Suspension mit einem Titer von $10^8$ $TCID_{50}/ml$ zugegeben. Nach Zusatz von 0.9 g Perlite J 100 und 0.9 g Celite 577 wurde 10 Minuten lang gerührt und anschliessexd durch ein Zellulosefilter filtriert. Titration von HAV im Filtrat ergab total $1.0 \times 10^6$ und $1.9 \times 10^5$ $TCID_{50}$ (geometrisches Mittel: $4.4 \times 10^5$). Der Abreicheruxgsfaktor betrug somit $2.3 \times 10^2$.

**Beispiel 5**

35 ml einer Fraktion von humanen Plasmaproteinen, welche den Gerinnungsfaktor VIII in hoher Konzentration enthielt, wurde mit 0.5 ml einer Suspension von HAV (Titer: $10^8$ $TCID_{50}/ml$) versetzt. Nach Zugabe von 0.11 g Bentonit und 30 Minuten Rühren wurde durch ein Zellulosefilter filtriert. Im Filtrat wurden total $7.9 \times 10^5$ und $6.3 \times 10^5$ $TCID_{50}$ HAV gemessen (geometrisches Mittel: $7.0 \times 10^5$), was einer Abreicherung um den Faktor $7.0 \times 10^1$ entspricht.

**Beispiel 6**

Je vier 50 ml Aliquots von Immunglobulin G Lösungen wurden auf pH 7 bzw 5 eingestellt. Anschliessend wurden die Lösungen mit 1 ml einer Suspension eines von vier Viren versetzt. Nach Zugabe von je 0.1 g Celite 577 wurde 16 Stunden gerührt, durch ein Zellulosefilter filtriert und die Viren wurden titriert:

| pH 7 | | | | | |
|---|---|---|---|---|---|
| Virus | Titer vor Filtration | Titer nach Filtration | | Geometrisches Mittel | Abreicherungs-faktor |
| SFV | 1.0E+10 | 7.5E+6 | 8.9E+6 | 8.2E+6 | 1.2E+3 |
| Sindbis | 1.0E+12 | 4.2E+8 | 1.9E+8 | 2.8E+8 | 3.5E+3 |
| VSV | 1.0E+10 | 2.0E+7 | 4.8E+7 | 3.1E+7 | 3.2E+2 |
| Coxsackie | 1.0E+7 | <10 | | | >1.0E+6 |
| | | | | | |
| pH 5 | | | | | |
| Virus | | | | | |
| SFV | 1.0E+10 | 6.9E+5 | 1.2E+5 | 2.9E+5 | 3.5E+4 |
| Sindbis | 1.0E+12 | 4.8E+5 | 1.1E+5 | 2.3E+5 | 4.4E+6 |
| VSV | 1.0E+10 | 2.1E+3 | 5.5E+3 | 3.4E+3 | 2.9E+6 |
| Coxsackie | 1.0E+7 | <10 | | | >1.0E+6 |

In den meisten Fällen ist der Abreicherungsfaktor bei tieferem pH grösser als bei höherem pH.

**Beispiel 7**

Je 50 ml Immunglobulinlösung wurden mit 1 ml Virussuspension versetzt. Anschliessend wurden je 0.1 g Celite 577 dazugegeben und nach 15 Minuten Rühren wurde durch ein Zellulosefilter filtriert. Im Filtrat wurden die Viren durch Titration bestimmt. Alle Versuche wurden sowohl bei 4°C als auch bei 20°C durchgeführt:

5

|  | 20 °C | | | 4 °C | |
|---|---|---|---|---|---|
| Virus | Titer vor Filtration | Titer nach Filtration | Abreicherungsfaktor | Titer nach Filtration | Abreicherungsfaktor |
| SFS | 1.0E + 10 | 7.5E + 6 | 1.3 E + 3 | 8.9 E + 6 | 1.1 E + 3 |
| Sindbis | 1.0E + 12 | 4.3E + 8 | 2.4 E + 3 | 1.1 E + 8 | 9.1 E + 3 |
| VSV | 1.0E + 10 | 4.8E + 7 | 2.1 E + 2 | 2.5 E + 7 | 4.0 E + 2 |

Innerhalb der für die Titration zu erwartenden Genauigkeit ist die Abreicherung durch Filtration im untersuchten Bereich unabhängig von der Temperatur.

**Beispiel 8**

Zu 50 ml Immunglobulinlösung wurde 1 ml einer Suspension von SFV zugegeben. Anschliessend wurden 0.1 g Celite eingerührt; nach weiteren 15 Minuten Rühren wurde durch ein Zellulosefilter filtriert. Der SFV Titer wurde im Filtrat durch Titration bestimmt. Zugabe von Celite, Rühren, Filtration und Bestimmung von Virus wurden noch zweimal mit dem Filtrat wiederholt:

|  | Titer vor Filtration | Titer nach Filtration | Abreicherungsfaktor |
|---|---|---|---|
| 1. Filtration | 1.0E + 10 | 1.2 E + 7 | 8.3 E + 2 |
| 2. Filtration | 1.2 E + 7 | 9.4 E + 5 | 1.3 E + 1 |
| 3. Filtration | 9.4 E + 5 | 1.2 E + 4 | 7.8 E + 1 |

Das Beispiel zeigt, dass durch mehrmalige Filtration aus derselben Lösung wiederholt Viren entfernt werden können.

**Patentansprüche**

1. Verfahren zur Abtrennung von Viren aus Proteinlösungen umfassend ein Suspendieren oder Aufschlämmen von Partikeln einer festen Phase zwecks Adsorption der Viren und eine anschliessende Trennung der festen Phase mit den daran adsorbierten Viren von der flüssigen Phase dadurch gekennzeichnet, dass die Proteinlösung mindestens einmal mit einer festen Phase in Form mindestens eines Adsorbens, ausgewählt aus der Gruppe Kieselgur, Kieselsäure, Tonmineralien, Metallhydroxid, Metalloxihydrat, Cellulose, Perlit und wasserunlösliche synthetische Polymere, während mindestens 10 min. unter Bildung einer Suspension oder Aufschlämmung in Kontakt gebracht und anschliessend vom Adsorbens getrennt wird.

2. Verfahren gemäss Anspruch 1, dadurch gekennzeichnet, dass die Trennung der festen Phase von der Proteinlösung durch Zentrifugation oder Filtration erfolgt.

3. Verfahren gemäss Anspruch 1, dadurch gekennzeichnet, dass die Proteinlösung ein Präparat aus humanem Blutplasma ist.

4. Verfahren gemäss einem der Ansprüche 1 bis 3, dadurch gekennzeichnet, dass die feste Phase ein unlösliches Metallhydroxid oder -oxihydrat, beispielsweise Magnesium- oder Aluminiumhydroxid ist

5. Verfahren gemäss Anspruch 4, dadurch gekennzeichnet, dass die feste Phase Aluminiumhydroxidgel enthält.

6. Verfahren gemäss einem der Ansprüche 1 bis 3, dadurch gekennzeichnet, dass die feste Phase Cellulose enthält.

7. Verfahren gemäss einem der Ansprüche 1 bis 3, dadurch gekennzeichnet, dass die feste Phase Diatomeenerde, Perlit, Puffperlit, Bentonit oder Talk ist.

8.  Verfahren gemäss einem der Ansprüche 1 bis 3, dadurch gekennzeichnet, dass die feste Phase in Form eines Gels vorliegt.

9.  Verfahren gemäss einem der Ansprüche 1 bis 8, dadurch gekennzeichnet, dass das Inkontaktbringen der Proteinlösung mit der festen Phase und das anschliessende Trennen der Phasen mindestens zweimal erfolgt, wobei ein Ersatz der festen Phase stattfindet.

10. Verfahren gemäss Anspruch 9, dadurch gekennzeichnet, dass das Inkontaktbringen der Proteinlösung mit der festen Phase und das anschliessende Trennen der Phasen mindestens dreimal erfolgt, wobei jeweils ein Ersatz der festen Phase stattfindet.

11. Verfahren gemäss einem der Ansprüche 1 bis 10, dadurch gekennzeichnet, dass der pH-Wert der Proteinlösung zwischen 4 und 7 liegt.

12. Verfahren gemäss Anspruch 11, dadurch gekennzeichnet, dass der pH-Wert und/oder die Ionenstärke der Proteinlösung während ihrem Kontakt mit der festen Phase mindestens einmal geändert wird.

13. Verfahren gemäss Anspruch 11, dadurch gekennzeichnet, dass der pH-Wert gesenkt wird.

14. Verfahren gemäss einem der Ansprüche 1 bis 13, dadurch gekennzeichnet, dass der Kontakt der Proteinlösung mit der festen Phase während 3 h bis 48 h erfolgt.

15. Verfahren gemäss einem der Ansprüche 1 bis 13, dadurch gekennzeichnet, dass der Kontakt der Proteinlösung mit der festen Phase während 30 min bis 12 h erfolgt.

| | Europäisches Patentamt | **EUROPÄISCHER RECHERCHENBERICHT** | Nummer der Anmeldung<br>EP 94 81 0231 |
|---|---|---|---|

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | WO-A-84 00569 (AMF)<br>* Ansprüche 20,21,47,49,50; Beispiel 5 *<br>--- | 1 | A61L2/00 |
| A | EP-A-0 085 917 (AMF)<br>* Anspruch 47; Beispiel 5 *<br>--- | 1 | |
| A | WO-A-91 10507 (OCTAPHARMA)<br>--- | | |
| A | US-A-4 305 870 (LIU D.T.H.)<br>----- | | |

**RECHERCHIERTE SACHGEBIETE (Int.Cl.6)**

A61L

Der vorliegende Recherchenbericht wurde für alle Patentansprüche erstellt

| Recherchenort | Abschlußdatum der Recherche | Prüfer |
|---|---|---|
| DEN HAAG | 18. Oktober 1994 | Peltre, C |

EPO FORM 1503 03.82 (P04C03)